# EUROPEAN PATENT SPECIFICATION

(11) **EP 2 915 884 B1**
(45) Date of publication and mention of the grant of the patent: **06.12.2017**
(21) Application number: 14382074.4
(22) Date of filing: 03.03.2014
(51) Int. Cl.: C12Q 1/68

(54) **Method for diagnosing colorectal cancer from a human feces sample by quantitive PCR**
Verfahren zur Diagnose von Kolorektalkarzinomen aus menschlichen Fäkalienproben mittels quantitativer Polymerasekettenreaktion
Procédé pour diagnostiquer le cancer colorectal à partir d'un échantillon de matières fécales humaines par PCR quantitative

(43) Date of publication of application: 09.09.2015
(73) Proprietor: Fundacio Institut d'Investigació Biomèdica de Girona Dr. Josep Trueta, 17190 Salt (Girona) (ES); Universitat de Girona, 17004 Girona (ES)
(72) Inventor: García-Gil, Jesús, 17833 Fontcoberta (Girona) (ES); Aldeguer, Xavier, 17003 Girona (ES); Serra Pagès, Mariona, 17600 Figueres (Girona) (ES); Mas de Xaxars, Teresa, 17833 Fontcoberta (Girona) (ES)
(74) Representative: Hoffmann Eitle

(56) References cited:
- WO-A2-2012/170478
- DATABASE EMBL [Online] 3 September 2009 (2009-09-03), "Uncultured bacterium isolate DGGE gel band Eub_3 16S ribosomal RNA gene, partial sequence.", XP002728405, retrieved from EBI accession no. EM_STD:GQ411111 Database accession no. GQ411111
- DATABASE EMBL [Online] 3 September 2009 (2009-09-03), "Uncultured bacterium isolate DGGE gel band Eub_10 16S ribosomal RNA gene, partial sequence.", XP002728406, retrieved from EBI accession no. EM_STD:GQ411118 Database accession no. GQ411118
- DATABASE EMBL [Online] 3 September 2009 (2009-09-03), "Uncultured bacterium isolate DGGE gel band Eub_46 16S ribosomal RNA gene, partial sequence.", XP002728407, retrieved from EBI accession no. EM_STD:GQ411150 Database accession no. GQ411150
- DATABASE EMBL [Online] 3 September 2009 (2009-09-03), "Uncultured bacterium isolate DGGE gel band Eub_48 16S ribosomal RNA gene, partial sequence.", XP002728408, retrieved from EBI accession no. EM_STD:GQ411152 Database accession no. GQ411152
- NA WU ET AL: "Dysbiosis Signature of Fecal Microbiota in Colorectal Cancer Patients", MICROBIAL ECOLOGY, vol. 66, no. 2, 4 June 2013 (2013-06-04), pages 462-470, XP055133734, ISSN: 0095-3628, DOI: 10.1007/s00248-013-0245-9
- WEIGUANG CHEN ET AL: "Human intestinal lumen and mucosa-associated microbiota in patients with colorectal cancer, art e39743", PLOS ONE, PUBLIC LIBRARY OF SCIENCE, US , vol. 7, no. 6 1 June 2012 (2012-06-01), pages 1-9, XP003031297, ISSN: 1932-6203, DOI: 10.1371/JOURNAL.PONE.0039743 Retrieved from the Internet: URL:http://www.plosone.org/article/info%3A doi%2F10.1371%2Fjournal.pone.0039743 [retrieved on 2012-06-28]
- Hassan Brim ET AL: "Microbiome Analysis of Stool Samples from African Americans with Colon Polyps", PLoS ONE, vol. 8, no. 12, 20 December 2013 (2013-12-20), page e81352, XP55188048, DOI: 10.1371/journal.pone.0081352
- Teresa Mas De Xaxars Rivero ET AL: "Descripció i quantificació de la microbiota intestinal associada al càncer colorectal", , 10 February 2012 (2012-02-10), XP55188205, Retrieved from the Internet: URL:http://hdl.handle.net/10803/94513 [retrieved on 2015-05-08]
- Patricia Lepage ET AL: "Biodiversity of the Mucosa-Associated Microbiota Is Stable Along the Distal Digestive Tract in Healthy Individuals and Patients With Ibd", Inflammatory Bowel Diseases, vol. 11, no. 5, 1 May 2005 (2005-05-01), pages 473-480, XP55187730, ISSN: 1078-0998, DOI: 10.1097/01.MIB.0000159662.62651.06

### SEQUENCE LISTING

<110> Institut dâ\200\231InvestigaciÃ³ BiomÃ¨dica de Girona Dr. Josep Trueta (IDIBGI)
   Universitat de Girona (UdG)
<120> METHOD FOR DIAGNOSING COLORECTAL CANCER FROM A HUMAN FECES SAMPLE BY QUANTITIVE PCR, PRIMERS AND KIT
<160> 12
<170> BiSSAP 1.2
<210> 1
   <211> 489
   <212> DNA
   <213> Artificial Sequence
<220>
   <221> source
   <222> 1..489
   <223> /mol_type="unassigned DNA" /note="B3 16S ribosomal DNA sequence, partial sequence (accesion number GQ411111.1)" /organism="Artificial Sequence"
<400> 1
   acggaggcct tcgggtcgta accgctttca gcagggaaga gtcaagactg tacctgcaga      60
   agaagccccg gctaactacg tgccagcagc cgcggtaata cgtagggggc gagcgttatc     120
   cggattcatt gggcgtaaag cgcgcgtagg cggcccggca ggccgggggt cgaagcgggg     180
   ggctcaaccc cccgaagccc ccggaacctc cgcggcttgg gtccggtagg ggagggtgga     240
   acacccggtg tagcggtgga atgcgcagat atcgggtgga acaccggtgg cgaaggcggc     300
   cctctgggcc gagaccgacg ctgaggcgcg aaagctgggg gagcgaacag gattagatac     360
   cctggtagtc ccagccgtaa acgatggacg ctaggtgtgg ggggacgatc cccccgtgcc     420
   gcagccaacg cattaagcgt cccgcctggg gagtacggcc gcaaggctaa aactcaaagg     480
   aattgacgg                                                             489
<210> 2
   <211> 19
   <212> DNA
   <213> Artificial Sequence
<220>
   <221> source
   <222> 1..19
   <223> /mol_type="unassigned DNA" /note="B3 16S ribosomal DNA sequence forward primer" /organism="Artificial Sequence"
<400> 2
   ggaggccttc gggtcgtaa 19
<210> 3
   <211> 18
   <212> DNA
   <213> Artificial Sequence
<220>
   <221> source
   <222> 1..18
   <223> /mol_type="unassigned DNA" /note="B3 16S ribosomal DNA sequence reverse primer" /organism="Artificial Sequence"
<400> 3
   ccgaagcccc cggaacct 18
<210> 4
   <211> 485
   <212> DNA
   <213> Artificial Sequence
<220>
   <221> source
   <222> 1..485
   <223> /mol_type="unassigned DNA" /note="B10 16S ribosomal DNA sequence, partial sequence (accesion number GQ411118.1)" /organism="Artificial Sequence"
<400> 4
   cttcggattg taaactcctg ttgttgagga gataatgacg gtactcaaca aggtaagtga      60
   cggctaacta cgtgccagca gccgcggtaa aacgtaggtc acaagcgttg tccggaatta     120
   ctgggtgtaa agggagcgca ggcgggaaga caagttggaa gtgaaatcca tgggctcaac     180
   ccatgaactg ctttcaaaac tgtttttctt gagtagtgca caggtaggcg gaattcccgg     240
   tgtagcggtg gaatgcgtag atatcgggag gaacaccagt ggcgaaggcg gcctactggg     300
   caccaactga cgctgaggct cgaaagtgtg ggtagcaaac aggattagat accctggtag     360
   tccacactgt aaacgatgat tactaggtgt tggaggattg accccttcag tgccgcagtt     420
   aacacaataa gtaatccacc tggggagtac gaccgcaagg ttgaaactca aaggaattga     480
   cggac                                                                 485
<210> 5
   <211> 19
   <212> DNA
   <213> Artificial Sequence
<220>
   <221> source
   <222> 1..19
   <223> /mol_type="unassigned DNA" /note="B10 16S ribosomal DNA sequence forward primer" /organism="Artificial Sequence"
<400> 5
   caacaaggta agtgacggc 19
<210> 6
   <211> 20
   <212> DNA
   <213> Artificial Sequence
<220>
   <221> source
   <222> 1..20
   <223> /mol_type="unassigned DNA" /note="B10 16S rDNA sequence reverse primer" /organism="Artificial Sequence"
<400> 6
   cgcctacctg tgcactactc 20
<210> 7
   <211> 474
   <212> DNA
   <213> Artificial Sequence
<220>
   <221> source
   <222> 1..474
   <223> /mol_type="unassigned DNA" /note="B46 16S ribosomal DNA sequence, partial sequence (accesion number GQ411150.1)" /organism="Artificial Sequence"
<400> 7
   gtaacatttc tgtaacaaga catacagcag tttctcagag ttcccaaact caaaggaatt    60
   gacggaagcc gcggttccac gtaagtcaca agcgttgtcc ggaattactg ggtgtaaagg   120
   gagcgcaggc gggaagacaa gttggaagtg aaatccatgg gctcaaccca taaactgctt   180
   tcctaactgt ttttcttgag tagtgcacag gtaggcggaa ttcccggtgt agcggtggaa   240
   tgcctagata tcgggaggaa caccattggc gaaggcggcc tactggactg caactgacgc   300
   tgaggctcga aagtgtgggt agcaaacagg attagatacc ctggtagtcc actccgtaaa   360
   ccatgattac tacgtgttgg aggattgacc ccttctgtgc cgcagataac acaataagta   420
   atccacctgg ggagtacgac cgcccggttg agactcacag gaattgacgg actc         474
<210> 8
   <211> 19
   <212> DNA
   <213> Artificial Sequence
<220>
   <221> source
   <222> 1..19
   <223> /mol_type="unassigned DNA" /note="B46 16S ribosomal DNA sequence forward primer" /organism="Artificial Sequence"
<400> 8
   tccacgtaag tcacaagcg 19
<210> 9
   <211> 20
   <212> DNA
   <213> Artificial Sequence
<220>
   <221> source
   <222> 1..20
   <223> /mol_type="unassigned DNA" /note="B46 16S ribosomal DNA sequence reverse primer" /organism="Artificial Sequence"
<400> 9
   cgcctacctg tgcactactc 20
<210> 10
   <211> 566
   <212> DNA
   <213> Artificial Sequence
<220>
   <221> source
   <222> 1..566
   <223> /mol_type="unassigned DNA" /note="B48 16S ribosomal DNA sequence, partial sequence (accesion number GQ411152.1)" /organism="Artificial Sequence"
<400> 10
   tctcttacgg ggagcagcag tggggaatat tgcacaatgg gggaaaccct gatgcagcga 60
   cgccgcgtga gcgatgaagt atttcggtat gtaaagctct atcagcaggg aagaaaatga 120
   cggtacctga ctaagaagcc ccggctaaat acgtgccagc agccgcggta atacgtatgg 180
   tgcaagcgtt atccggattt actgggtgta aagggagcgt agacggagtg gcaagtctga 240
   tgtgaaaacc cggggctcaa ccccgggact gcattggaaa ctgtgcatct agagtgtcgg 300
   agaggtaagc ggaattccta gtgtagcggt gaaatgcgta gatattagga ggaacaccag 360
   tggcgaaggc ggcttactgg acgataactg acgctgaggc tcgaaagcgt ggggagcaaa 420
   caggattaga taccctggta gtccacgccg taaacgatga ctactaggtg tcggggagca 480
   cagctcttcg gtgccgcagc aaacgcaata agtattccac ctggggagta cgttcgcaag 540
   aatgaaactc acagaatttg acggag 566
<210> 11
   <211> 18
   <212> DNA
   <213> Artificial Sequence
<220>
   <221> source
   <222> 1..18
   <223> /mol_type="unassigned DNA" /note="B48 16S ribosomal DNA sequence forward primer" /organism="Artificial Sequence"
<400> 11
   gtacggggag cagcagtg 18
<210> 12
   <211> 23
   <212> DNA
   <213> Artificial Sequence
<220>
   <221> source
   <222> 1..23
   <223> /mol_type="unassigned DNA" /note="B48 16S ribosomal DNA sequence reverse primer" /organism="Artificial Sequence"
<400> 12
   gacactctag atgcacagtt tcc 23

## Description

### FIELD OF THE INVENTION

The present invention relates to molecular diagnostic methods. Specifically, in the present invention, colorectal cancer is diagnosed from the quantification by PCR of specific bacterial DNA sequences present in the feces.

### BACKGROUND OF THE INVENTION

Colorectal cancer (CRC) is the second leading cause of cancer death in Europe and in United States, and is the most frequently diagnosed cancer in Europe, with over 400,000 new cases and 200,000 deaths in 2008.

These data reflect that preventive measures need to be taken. The most effective and economic measure to reduce CRC incidence and mortality are CRC risk screening test, thus an improvement of the sensitivity from actual screening methods is required. For this reason, it is crucial a better understanding of the CRC etiology to find the key biomarker for CRC risk screening.

The genetic basis and natural history of CRC are well defined, considering that less than 5 % of CRCs are hereditary, and majority are sporadic, diagnosed in patients with no personal or family history of colonic neoplasm. The etiology of this disease is still unknown, although a multifactorial origin in which endogenous and exogenous factors are actively involved in tumor development is suspected. These factors are: age, tobacco, personal history of inflammatory bowel disease, diet, lifestyle, and more recently microbiota.

Lately, it has been shown that bacterial communities in the colonic mucosa of CRC patients differ from healthy individuals and the intestinal microbiota has been proposed as a determining agent in the development and progression of CRC along its stages (Chen W et al. Human intestinal lumen and mucosa-associated microbiota in patients with colorectal cancer. PLoS One. 2012;7(6):e39743; Zhu Q et al. The role of gut microbiota in the pathogenesis of colorectal cancer. Tumour Biol. 2013 Jun;34(3):1285-300; Ahn J et al. Human gut microbiome and risk for colorectal cancer. J Natl Cancer Inst. 2013 Dec 18;105(24):1907-11).

Na wu et al (Microbial ecology, 2013;66(2):462-470) describes the observed differences in fecal microbiota composition between healthy individuals and CRC patients. It mentions the possible use of *Fusobacterium spp.* as fecal marker for the pre-diagnosis of CRC. It does not provide however any specificity/sensitivity data actually confirming the usefulness of *Fusobacterium spp.* as a biomarker of CRC diagnosis in fecal samples.

On the other hand, Hassan Brim et al (PLoS ONE, 8(12), e81352) refers to the taxonomic profiling of bacterial sequences in feces samples from healthy individuals and patients with colon polyps using 16S rRNA gene microarray technology and pyrosequencing. The presence of colon polyps is said to be associated with a 10% risk of malignant progression. No differences in microbiota composition are reported at the genus level between the two groups. At the sub-genus level bacteria from the Bacteroides group were found to be predominant in the polyp patient's samples. Nevertheless, the metagenomics analysis did not reveal major differences in bacterial gene prevalence/abundances between the two groups even when the analysis and comparisons were restricted to available Bacteroides genomes.

Teresa Mas de Xaxars. (2012, Dipòsit legal: GI. 1664-2012, http://hdl.handle.net/10803/94513) discloses an association in intestinal mucosal samples between CRC and prevalence of the bacterial sequences SEQ ID NO:1, SEQ ID NO:4, SEQ ID NO:7, and SEQ ID NO:10 of the present invention as determined by denaturating gradient gel electrophoresis (DGGE). This document does not teach however an association between abundance of these sequences and CRC diagnosis. Moreover, the observed relationship is in mucosal samples, not in feces samples.

The problem to be solved can be seen in the provision of a reliable method for diagnosing colorectal cancer from human feces samples.

The present invention, as defined in the claims, provides a solution to this problem.

### DESCRIPTION OF THE INVENTION

In the present invention, specific bacterial sequences associated to diagnostic of colorectal cancer and diagnostic of risk of developing colorectal cancer have been identified.

One preferred embodiment is a method for diagnosing colorectal cancer from a human feces sample, comprising:
(a) determining by quantitative PCR in said human feces sample the concentration of at least one 16S rDNA bacterial sequences selected from the group consisting of SEQ ID NO: 4, SEQ ID NO: 7 and SEQ ID NO: 10, wherein SEQ ID NO: 4 concentration is determined using primers with at least 90% identity with respect to SEQ ID NO: 5-6, SEQ ID NO: 7 concentration is determined using primers with at least 90% identity with respect to SEQ ID NO: 8-9 and SEQ ID NO: 10 concentration is determined using primers with at least 90% identity with respect to SEQ ID NO: 11-12,
(b) optionally, determining at least one of the ratios of concentrations selected from the group consisting of SEQ ID NO: 10/SEQ ID NO: 4 and SEQ ID NO: 7/SEQ ID NO: 4, and
(c) diagnosing colorectal cancer from the concentration of at least one of the sequences of step (a) and/or the value of at least one ratio obtained in step (b); wherein colorectal cancer is diagnosed in step c) if there is an increase in the Ct value of at least one of the sequences of step (a) and/or a decrease in the value of at least one ratio obtained in step (b) with respect to a cut-off value, herewith, method for diagnosing colorectal cancer of the invention.

In the present application, the sequence identified by SEQ ID NO: 1 is also identified as "B3". Similarly, SEQ ID NO: 4 is also identified as "B10", SEQ ID NO: 7 is also identified as "B46" and SEQ ID NO: 10 is also identified as "B48". B3, B10, B46 and B48 were the names used by the inventors of the present application to identify the sequences during performance of all experiments carried out to arrive at the present invention.

The sequences identified by SEQ ID NO: 1, 4, 7 and 10 are available in public sequences databases under the following accesion numbers: GQ411111.1 (SEQ ID NO: 1), GQ411118.1 (SEQ ID NO: 4), GQ411150.1 (SEQ ID NO: 7), GQ411152.1 (SEQ ID NO: 10).

However, the presence of said sequences has not been identified in human feces samples and a method of diagnosing cancer colorectal cancer from a human feces sample based on the determination by quantitative PCR of the concentration of these sequences, using the primers defined in the claims of the present application, has not been suggested in the prior art.

In the present application, a problem sequence has 95% identity with respect to a determined sequence if 95% of residues of the problem sequence are identical to the residues of the determined sequence.

In the present application, the ratio of concentrations of the sequences SEQ ID NO: 10/SEQ ID NO: 4 is obtained by dividing the concentration of the sequence identified by SEQ ID NO: 10 by the concentration of the sequence identified by SEQ ID NO: 4. The ratio could also be obtained by dividing the Ct value of the sequence identified by SEQ ID NO: 10 by the Ct value of the sequence identified by SEQ ID NO: 4. The meaning of Ct is explained in Example 3.

The rest of the ratios indicated in the claims shall be calculated accordingly.

The method of the invention is a potent tool to prevent colorectal cancer through an early stage disease diagnostic.

Another embodiment is the method of the invention, wherein said primers with at least 90% identity with respect to SEQ ID NO: 5-6 are primers with at least 95% identity with respect to SEQ ID NO: 5-6,said primers with at least 90% identity with respect to SEQ ID NO: 8-9 are primers with at least 95% identity with respect to SEQ ID NO: 8-9 and said primers with at least 90% identity with respect to SEQ ID NO: 11-12 are primers with at least 95% identity with respect to SEQ ID NO: 11-12. Particularly, said primers with at least 95% identity with respect to SEQ ID NO: 5-6 are primers identified by SEQ ID NO: 5-6, said primers with at least 95% identity with respect to SEQ ID NO: 8-9 are primers identified by SEQ ID NO: 8-9 and said primers with at least 95% identity with respect to SEQ ID NO: 11-12 are primers identified by SEQ ID NO: 11-12.

Another embodiment is the method of the invention, wherein colorectal cancer is diagnosed in step (c) if the concentration of SEQ ID NO: 4 is above the cutoff Ct value of 16.56, or if the concentration of SEQ ID NO: 7 is above the cutoff Ct value of 22.97, or if the concentration of SEQ ID NO: 10 is above the cutoff Ct value of 19.24, or if the value of the ratio SEQ ID NO: 10/SEQ ID NO: 4 is below the cutoff value of 1.16, or if the value of the ratio SEQ ID NO: 7/SEQ ID NO: 4 is below the cutoff value of 1.40.

One goal of the present invention is to provide a pre-diagnosis tool. The present invention provides a method for diagnosing colorectal cancer before having clinical signs and to discern the high risk population.

Thus, another preferred embodiment of the present invention is a method for diagnosing the risk of developing colorectal cancer from a human feces sample, comprising:
(a) determining by quantitative PCR in said human feces sample the concentration of at least one 16S rDNA bacterial sequences selected from the group consisting of SEQ ID NO: 7 and SEQ ID NO: 10, wherein SEQ ID NO: 7 concentration is determined using primers with at least 90% identity with respect to SEQ ID NO: 8-9 and SEQ ID NO: 10 concentration is determined using primers with at least 90% identity with respect to SEQ ID NO: 11-12,
(b) optionally, determining at least one of the ratios of concentrations selected from the group consisting of SEQ ID NO: 10/SEQ ID NO: 4, and SEQ ID NO: 7/SEQ ID NO: 4; wherein SEQ ID NO: 4 concentration is determined by quantitative PCR in said human feces sample using primers with at least 90% identity with respect to SEQ ID NO: 5-6; and
(c) diagnosing colorectal cancer from the concentration of at least one of the sequences of step (a) and/or the value of at least one ratio obtained in step (b); wherein an increased risk of developing colorectal cancer is diagnosed in step c) if there is an increase in the Ct value of at least one of the sequences of step (a) and/or a decrease in the value of at least one ratio obtained in step (b) with respect to a cut-off value, herewith, method for diagnosing the risk of developing colorectal cancer of the invention.

Another embodiment is the method for diagnosing the risk of developing colorectal cancer, wherein a risk of developing colorectal cancer is diagnosed in step (c) if the concentration of SEQ ID NO: 7 is above the cutoff Ct value of 24.76, or if the concentration of SEQ ID NO: 10 is above the cutoff Ct value of 21.42, or if the value of the ratio SEQ ID NO: 10/SEQ ID NO: 4 is below the cutoff value of 1.56, or if the value of the ratio SEQ ID NO: 7/SEQ ID NO: 4 is below the cutoff value of 1.78.

It is also described herein a PCR primer identified by any of the sequences selected from the group consisting of SEQ ID NO: 5-6, SEQ ID NO: 8-9 and SEQ ID NO: 11-12

Another embodiment of the present invention relates to the use of a kit in a method for diagnosing colorectal cancer from a human feces sample according to the present invention, comprising at least one pair of PCR primers selected from the pair of primers group consisting of SEQ ID NO: 5-6, SEQ ID NO: 8-9 and SEQ ID NO: 11-12.

### BRIEF DESCRIPTION OF THE FIGURES

Figure 1. Ratio of Ct values for 2 sequences: SEQ ID NO: 10/SEQ ID NO: 4. CRC represents the values obtained from feces samples of cancer colorectal patients, and C represents the control values obtained from healthy patients.
Figure 2. Ratio of Ct values for 2 sequences: SEQ ID NO: 7/SEQ ID NO: 4. CRC represents the values obtained from feces samples of cancer colorectal patients, and C represents the control values obtained from healthy patients.
Figure 3. Ratio of Ct values for 2 sequences: SEQ ID NO: 4/SEQ ID NO: 1. CRC represents the values obtained from feces samples of cancer colorectal patients, and C represents the control values obtained from healthy patients.
Figure 4. Absolute Ct values for the sequence SEQ ID NO: 4. CRC represents the values obtained from feces samples of cancer colorectal patients, and C represents the control values obtained from healthy patients.
Figure 5. Absolute Ct values for the sequence SEQ ID NO: 7. CRC represents the values obtained from feces samples of cancer colorectal patients, and C represents the control values obtained from healthy patients.
Figure 6. Ratio Ct values for 2 sequences: SEQ ID NO: 4/ SEQ ID NO: 1. CRC represents the values obtained from feces samples of cancer colorectal cancer, High Risk represents values obtained from individuals with Lynch syndrome who had polyps in his last colonoscopy and have an increased risk of developing colorectal cancer, Low Risk represents values obtained from individuals with Lynch syndrome who had no polyps in his last colonoscopy.
Figure 7. Absolute Ct values for the sequence SEQ ID NO: 4. CRC represents the values obtained from feces samples of cancer colorectal cancer, High Risk represents values obtained from individuals with Lynch syndrome who had polyps in his last colonoscopy and have an increased risk of developing colorectal cancer, Low Risk represents values obtained from individuals with Lynch syndrome who had no polyps in his last colonoscopy.

### EXAMPLES OF THE INVENTION

Example 1: Patient samples and identification of bacterial markers.

In example 3, a total of 16 samples of feces in 7 controls and 9 colorectal cancer patients have been analyzed.

In example 4, a total of 8 individuals with Lynch Syndrome (with genetic increased risk to develop colorectal cancer) were analyzed.

All individuals had a colonoscopy, in the maximum period of one year, before the fecal sample collection. According to endoscopic exam 4 of them had had malign polyp (named as High Risk) and 4 had not any polyp (named as Low Risk).

The following bacterial markers have been identified in said human samples:
- 16S rDNA bacterial sequence SEQ ID NO: 1 (B3)
- 16S rDNA bacterial sequence SEQ ID NO: 4 (B10)
- 16S rDNA bacterial sequence SEQ ID NO: 7 (B46)
- 16S rDNA bacterial sequence SEQ ID NO: 10 (B48)

### Example 2. Primer design

The primers of the present disclosure (SEQ ID NO: 2-3, SEQ ID NO: 5-6, SEQ ID NO: 8-9 and SEQ ID NO: 11-12) have been designed from the comparative analysis with sequences previously obtained from phylogenetic groups using bioinformatics tools: ClustalX, Netprimer and PrimerExpress. Said primers have been designed for the quantification of the bacterial markers cited in Example 1 by quantitative PCR.

The detection system chosen was SybrGreen® or similar. Set of primers with less than 3 positions different with respect to groups nearby were discarded. Set of primers with Tm values in dissociation curves different of expected ones were also discarded.

### Example 3: Quantitative PCR methods and results in colorectal cancer patients

The quantification of each of the bacteria markers cited in Example 1 in feces samples analyzed is expressed in Ct values. The Ct (cycle threshold) is defined as the number of q-PCR cycles required for the fluorescent signal to cross the threshold. Ct levels are inversely proportional to the amount of target nucleic acid in the sample (ie the lower the Ct level the greater the amount of target nucleic acid in the sample). WVDL real time assays undergo 40 cycles of amplification.

The obtained results are shown below, in Tables 1 and 2. Table 1 represents Ct absolute values and Table 2 represents Ct ratios.

**Table 1. Ct absolute values**

| ID | Group | Condition | B48 | B46 | B10 | B3 |
|---|---|---|---|---|---|---|
| F10 | CRC | 1 | 18.59 | 22.78 | 16.33 | 33.64 |
| F11 | CRC | 1 | 20.33 | 26.45 | 20.37 | 41.57 |
| F3 | CRC | 1 | 23.52 | 26.2 | 19.8 | 38.45 |
| F4 | CRC | 1 | 16.41 | 20.87 | 14.88 | |
| F5 | CRC | 1 | 19.04 | 24 | 17.61 | 35.68 |
| F6 | CRC | 1 | 23.22 | 27.67 | 21.82 | |
| F16 | CRC | 1 | | 20.75 | 14.35 | |
| F7 | CRC | 1 | 23.19 | 28.2 | 22.34 | |
| F8 | CRC | 1 | 18.82 | 22.04 | 15.61 | |
| F12 | C | 0 | 17.55 | 20.9 | 14.95 | |
| F13 | C | 0 | 19.36 | 24.78 | 19.22 | |
| F14 | C | 0 | 19.46 | 21.44 | 15.07 | 37.34 |
| F15 | C | 0 | 17.55 | 19.85 | 12.84 | 36.98 |
| F9 | C | 0 | 17.61 | 21.8 | 15.41 | 35.66 |
| F1 | C | 0 | 18.09 | 21.97 | 14.79 | 38.3 |
| F2 | C | 0 | 17.07 | 20.57 | 12.76 | |
| Mean | | CRC | 20.39 | 24.329 | 18.12 | 37.34 |
| | | C | 18.099 | 21.616 | 15.01 | 37.07 |
| Standard deviation | | CRC | 2.65 | 2.89 | 3.04 | 3.44 |
| | | C | 0.94 | 1.58 | 2.15 | 1.09 |
| P-value | | CRC vs C | 0.025 | 0.021 | 0.019 | 0.444 |
| Mid-point (cutoff values) | | | 19.244 | 22.972 | 16.56 | 37.2 |
| Sensitivity % | | | 66.67 | 83.33 | 83.33 | 50 |
| Specificity % | | | 55.56 | 60 | 60 | 50 |
| Accuracy | | | 60 | 68.75 | 68.75 | 50 |

**Table 2. Ct ratios**

| ID | Group | Condition | B48/B10 | B10/B3 | B46/B10 | B46/B48 | B46/B3 | B48/B3 |
|---|---|---|---|---|---|---|---|---|
| F10 | CRC | 1 | 1.14 | 0.49 | 1.39 | 1.23 | 0.68 | 0.55 |
| F11 | CRC | 1 | 1 | 0.49 | 1.3 | 1.3 | 0.64 | 0.49 |
| F3 | CRC | 1 | 1.19 | 0.51 | 1.32 | 1.11 | 0.68 | 0.61 |
| F4 | CRC | 1 | 1.1 | | 1.4 | 1.27 | | |
| F5 | CRC | 1 | 1.08 | 0.49 | 1.36 | 1.26 | 0.67 | 0.53 |
| F6 | CRC | 1 | 1.06 | | 1.27 | 1.19 | | |
| F16 | CRC | 1 | | | 1.45 | | | |
| F7 | CRC | 1 | 1.04 | | 1.26 | 1.22 | | |
| F8 | CRC | 1 | 1.21 | | 1.41 | 1.17 | | |
| F12 | C | 0 | 1.17 | | 1.4 | 1.19 | | |
| F13 | C | 0 | 1.01 | | 1.29 | 1.28 | | |
| F14 | C | 0 | 1.29 | 0.4 | 1.42 | 1.1 | 0.57 | 0.52 |
| F15 | C | 0 | 1.37 | 0.35 | 1.55 | 1.13 | 0.54 | 0.47 |
| F9 | C | 0 | 1.14 | 0.43 | 1.41 | 1.24 | 0.61 | 0.49 |
| F1 | C | 0 | 1.22 | 0.39 | 1.49 | 1.21 | 0.57 | 0.47 |
| F2 | C | 0 | 1.34 | | 1.61 | 1.21 | | |
| Mean | | CRC | 1.1 | 0.5 | 1.35 | 1.22 | 0.67 | 0.55 |
| | | C | 1.22 | 0.39 | 1.45 | 1.19 | 0.57 | 0.49 |
| Standard deviation | | CRC | 0.07 | 0.01 | 0.07 | 0.06 | 0.02 | 0.05 |
| | | C | 0.13 | 0.04 | 0.11 | 0.06 | 0.03 | 0.02 |
| P-value | | CRC vs C | 0.02 | 0.001 | 0.018 | 0.224 | 0.001 | 0.045 |
| Mid-point (cutoff values) | | | 1.16123 | 0.4441 | 1.40243 | 1.20669 | 0.62 | 0.52 |
| Sensitivity % | | | 75 | 100 | 77.78 | 55.56 | 100.00 | 75.00 |
| Specificity % | | | 71.43 | 100 | 71.43 | 42.86 | 100.00 | 75.00 |
| Accuracy | | | 73.33 | 100 | 75 | 50 | 100.00 | 75.00 |

The sensitivity and specificity of the diagnosis of colorectal cancer is 75 and 71% for the ratio SEQ ID NO: 10/SEQ ID NO: 4; 77% and 71%, respectively, for the ratio SEQ ID NO: 7/SEQ ID NO: 4; 100% and 100%, respectively, for the ratio SEQ ID NO: 4/SEQ ID NO: 1; 100% and 100%, respectively, for the ratio SEQ ID NO: 7/ SEQ ID NO: 1; and 75% and 75%, respectively, for the ratio SEQ ID NO: 10/ SEQ ID NO: 1.

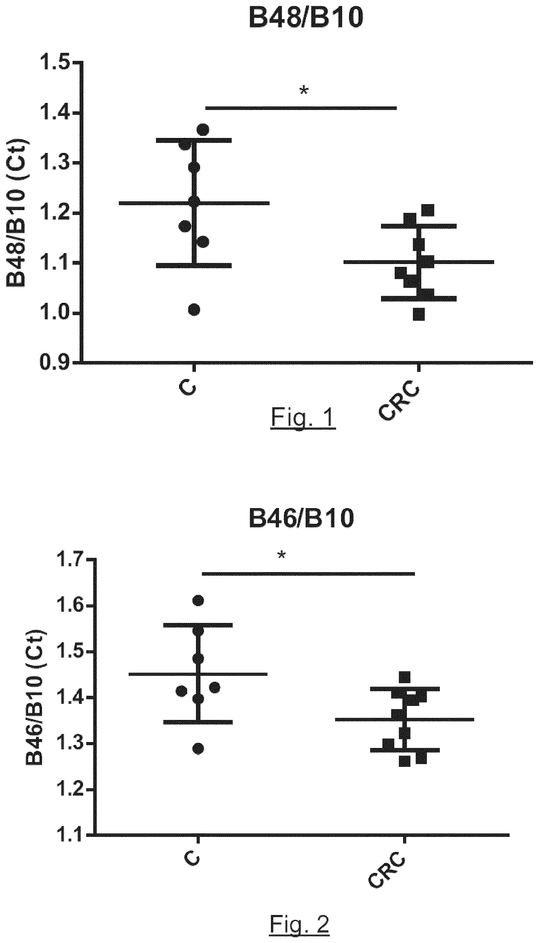

Figures 1, 2 and 3 are graphical representations of ratios of Ct values (SEQ ID NO: 10/SEQ ID NO: 4, SEQ ID NO: 7/SEQ ID NO: 4 and SEQ ID NO: 4/SEQ ID NO: 1).

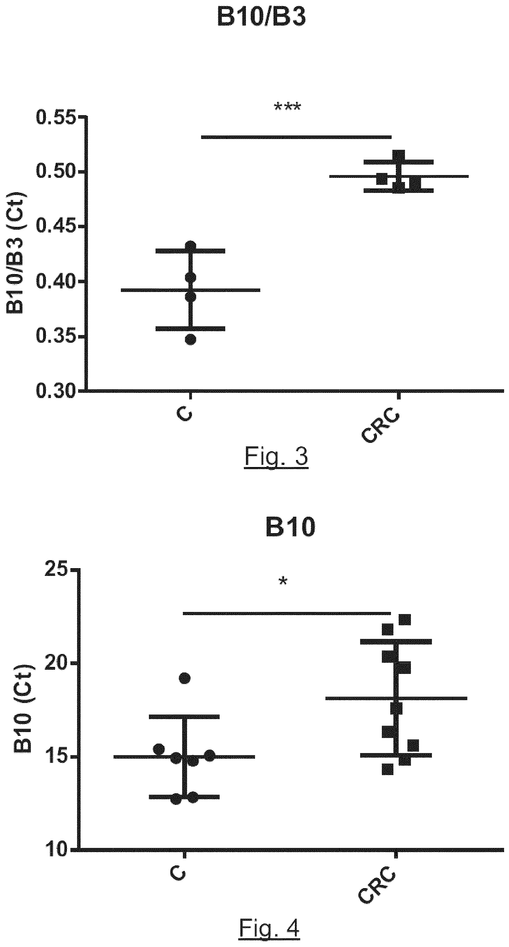

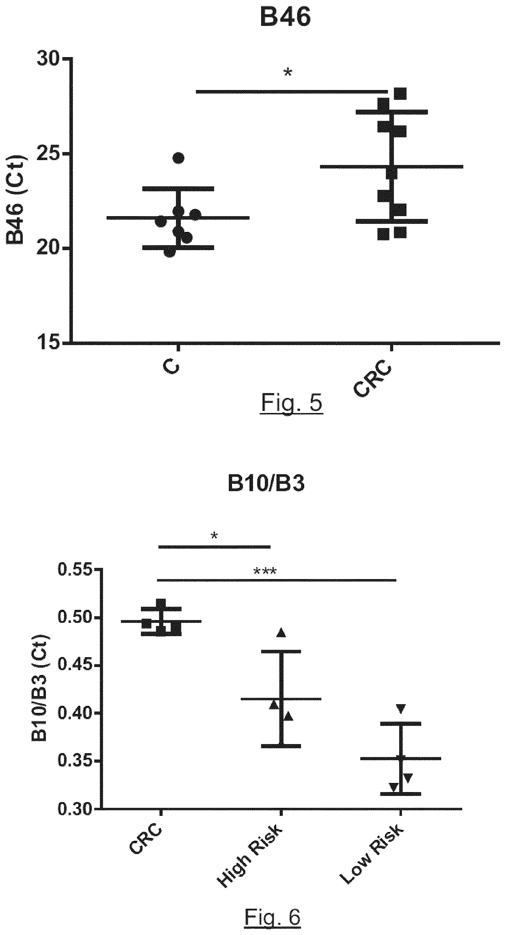

Figures 4 and 5 are graphical representations of the Ct absolutes (SEQ ID NO: 4, SEQ ID NO: 7).

### Example 4. Colorectal cancer risk before clinical signs. Quantitative PCR methods and results in Lynch syndrome patients

The quantification of each of the bacteria markers cited in Example 1 in human feces samples analyzed were determined using the primers described in Example 2 and were quantified in Ct values.

The obtained results are shown below, in Tables 3 and 4. Table 3 represents Ct absolute values and Table 4 represents Ct ratios.

**Table 3. Ct absolute values**

| ID | Group | Condition | B48 | B46 | B10 | B3 |
|---|---|---|---|---|---|---|
| F10 | CRC | 1 | 18.59 | 22.78 | 16.33 | 33.64 |
| F11 | CRC | 1 | 20.33 | 26.45 | 20.37 | 41.57 |
| F3 | CRC | 1 | 23.52 | 26.20 | 19.80 | 38.45 |
| F4 | CRC | 1 | 16.41 | 20.87 | 14.88 | |
| F5 | CRC | 1 | 19.04 | 24.00 | 17.61 | 35.68 |
| F6 | CRC | 1 | 23.22 | 27.67 | 21.82 | |
| F16 | CRC | 1 | | 20.75 | 14.35 | |
| F7 | CRC | 1 | 23.19 | 28.20 | 22.34 | |
| F8 | CRC | 1 | 18.82 | 22.04 | 15.61 | |
| MIL1 | High Risk | 2 | 22.58 | 25.87 | 14.56 | 35.56 |
| MIL2 | High Risk | 2 | 24.53 | 25.79 | 14.73 | 37.06 |
| MIL5 | High Risk | 2 | 22.56 | 24.57 | 14.16 | 38.44 |
| MIL4 | High Risk | 2 | 20.00 | 29.90 | 18.41 | 37.99 |
| MIL6 | Low Risk | 3 | 20.15 | 24.90 | 15.20 | 37.58 |
| MIL3 | Low Risk | 3 | 18.11 | 20.45 | 10.34 | 31.16 |
| MIL7 | Low Risk | 3 | 22.81 | 24.70 | 13.35 | 41.40 |
| MIL8 | Low Risk | 3 | 20.60 | 21.88 | 11.38 | 32.42 |
| Mean | | CRC | 20.39 | 24.33 | 18.12 | 37.34 |
| | | High Risk | 22.42 | 26.53 | 15.46 | 37.26 |
| | | Low Risk | 20.42 | 22.98 | 12.57 | 35.64 |
| Standard deviation | | CRC | 2.65 | 2.89 | 3.04 | 3.44 |
| | | High Risk | 1.86 | 2,32 | 1,98 | 1,27 |
| | | Low Risk | 1,93 | 2,18 | 2,15 | 4,74 |
| P-value | | CRC vs High Risk | 0,10 | 0,10 | 0,070 | 0,48 |
| | | CRC vs Low Risk | 0,49 | 0,21 | 0,004 | 0,29 |
| | | High Risk vs Low Risk | 0,09 | 0,03 | 0,047 | 0,27 |
| Mid-point (cutoff values) | | | 21,42 | 24,76 | 14,02 | 36,45 |
| Sensitivity % | | | 75,00 | 75,00 | 80,00 | 60,00 |
| Specificity % | | | 75,00 | 75,00 | 100,00 | 66,67 |
| Accuracy | | | 75,00 | 75,00 | 87,50 | 62,50 |

**Table 4. Ct ratios**

| ID | Group | Condition | B48/B10 | B10/B3 | B46/B10 | B46/B48 | B46/B3 | B48/B3 |
|---|---|---|---|---|---|---|---|---|
| F10 | CRC | 1 | 1.14 | 0.49 | 1.39 | 1.23 | 0.68 | 0.55 |
| F11 | CRC | 1 | 1.00 | 0.49 | 1.30 | 1.30 | 0.64 | 0.49 |
| F3 | CRC | 1 | 1.19 | 0.51 | 1.32 | 1.11 | 0.68 | 0.61 |
| F4 | CRC | 1 | 1.10 | | 1.40 | 1.27 | | |
| F5 | CRC | 1 | 1.08 | 0.49 | 1.36 | 1.26 | 0.67 | 0.53 |
| F6 | CRC | 1 | 1.06 | | 1.27 | 1.19 | | |
| F16 | CRC | 1 | | | 1.45 | | | |
| F7 | CRC | 1 | 1.04 | | 1.26 | 1.22 | | |
| F8 | CRC | 1 | 1.21 | | 1.41 | 1.17 | | |
| MIL1 | High Risk | 2 | 1.55 | 0.41 | 1.78 | 1.15 | 0.73 | 0.63 |
| MIL2 | High Risk | 2 | 1.67 | 0.40 | 1.75 | 1.05 | 0.70 | 0.66 |
| MIL5 | High Risk | 2 | 1.59 | 0.37 | 1.74 | 1.09 | 0.64 | 0.59 |
| MIL4 | High Risk | 2 | 1.09 | 0.48 | 1.62 | 1.49 | 0.79 | 0.53 |
| MIL6 | Low Risk | 3 | 1.33 | 0.40 | 1.64 | 1.24 | 0.66 | 0.54 |
| MIL3 | Low Risk | 3 | 1.75 | 0.33 | 1.98 | 1.13 | 0.66 | 0.58 |
| MIL7 | Low Risk | 3 | 1.71 | 0.32 | 1.85 | 1.08 | 0.60 | 0.55 |
| MIL8 | Low Risk | 3 | 1.81 | 0.35 | 1.92 | 1.06 | 0.67 | 0.64 |
| Mean | | CRC | 1.10 | 0.50 | 1.35 | 1.22 | 0.67 | 0.55 |
| | | High Risk | 1.47 | 0.41 | 1.72 | 1.20 | 0.71 | 0.60 |
| | | Low Risk | 1.65 | 0.35 | 1.85 | 1.13 | 0.65 | 0.58 |
| Standard deviation | | CRC | 0.07 | 0.01 | 0.07 | 0.06 | 0.02 | 0.05 |
| | | High Risk | 0.26 | 0.05 | 0.07 | 0.20 | 0.06 | 0.06 |
| | | Low Risk | 0.22 | 0.04 | 0.15 | 0.08 | 0.03 | 0.04 |
| P-value | | CRC vs High Risk | 0.001491 | 0.009755 | 0.000001 | 0.38 | 0.11 | 0.10 |
| | | CRC vs Low Risk | 0.000029 | 0.000159 | 0.000002 | 0.02 | 0.19 | 0.21 |
| | | High Risk vs Low Risk | 0.172991 | 0.044382 | 0.087799 | 0.28 | 0.06 | 0.25 |
| Mid-point (cutoff values) | | | 1.56 | 0.38 | 1.78 | 1.16 | 0.68 | 0.59 |
| Sensitivity % | | | 66.67 | 75.00 | 80.00 | 50.00 | 75.00 | 75.00 |
| Specificity % | | | 60.00 | 75.00 | 100.00 | 50.00 | 75.00 | 75.00 |
| Accuracy | | | 62.50 | 75.00 | 87.50 | 50.00 | 75.00 | 75.00 |

The microbiological ratio markers were able to discern the group of individuals with High Risk to develop colorectal cancer (patients with Lynch syndrome and polyps). The sensitivity and specificity in detecting the colorectal cancer high risk population were 80% and 100% for the SEQ ID NO: 4; 75% and 75%, respectively, for the ratio SEQ ID NO: 4/SEQ ID NO: 1; 80% and 100%, respectively, for the ratio SEQ ID NO: 7/SEQ ID NO: 4; 75% and 75%, respectively, for the ratio SEQ ID NO: 7/SEQ ID NO: 1; and 75% and 75% for the ratio SEQ ID NO: 10/SEQ ID NO: 1.

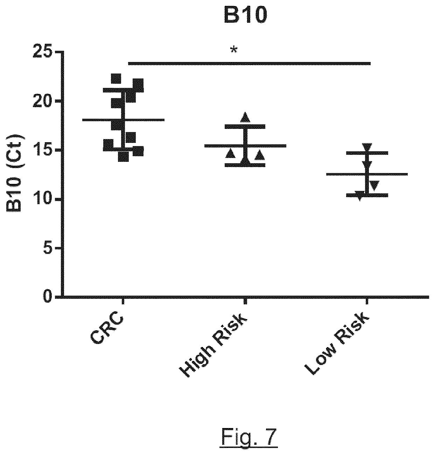

Figure 6 is graphical representation of ratio of Ct values (SEQ ID NO: 4/SEQ ID NO: 1) and Figure 7 is a graphical representation of the Ct absolutes (SEQ ID NO: 4).

## Claims

1. Method for diagnosing the risk of developing colorectal cancer from a human feces sample, comprising:
(a) determining by quantitative PCR in said human feces sample the concentration of at least one 16S rDNA bacterial sequence selected from the group consisting of SEQ ID NO: 7 and SEQ ID NO: 10,
wherein SEQ ID NO: 7 concentration is determined using primers with at least 90% identity with respect to SEQ ID NO: 8-9, and SEQ ID NO: 10 concentration is determined using primers with at least 90% identity with respect to SEQ ID NO: 11-12;
(b) optionally, determining at least one of the ratios of concentrations selected from the group consisting of SEQ ID NO: 10/SEQ ID NO: 4, and SEQ ID NO: 7/SEQ ID NO: 4, wherein SEQ ID NO: 4 concentration is determined by quantitative PCR in said human feces sample using primers with at least 90% identity with respect to SEQ ID NO: 5-6; and
(c) diagnosing the risk of developing colorectal cancer from the concentration of at least one of the sequences of step (a) and/or the value of at least one ratio obtained in step (b);
wherein an increased risk of developing colorectal cancer is diagnosed in step c) if there is an increase in the Ct value of at least one of the sequences of step (a) and/or a decrease in the value of at least one ratio obtained in step (b) with respect to a cut-off value.

2. Method for diagnosing colorectal cancer from a human feces sample, comprising:
(a) determining by quantitative PCR in said human feces sample the concentration of at least one 16S rDNA bacterial sequence selected from the group consisting of SEQ ID NO: 4, SEQ ID NO: 7 and SEQ ID NO: 10,
wherein SEQ ID NO: 4 concentration is determined using primers with at least 90% identity with respect to SEQ ID NO: 5-6, SEQ ID NO: 7 concentration is determined using primers with at least 90% identity with respect to SEQ ID NO: 8-9, and SEQ ID NO: 10 concentration is determined using primers with at least 90% identity with respect to SEQ ID NO: 11-12;
(b) optionally, determining at least one of the ratios of concentrations selected from the group consisting of SEQ ID NO: 10/SEQ ID NO: 4, and SEQ ID NO: 7/SEQ ID NO: 4; and
(c) diagnosing colorectal cancer from the concentration of at least one of the sequences of step (a) and/or the value of at least one ratio obtained in step (b);
wherein colorectal cancer is diagnosed in step c) if there is an increase in the Ct value of at least one of the sequences of step (a) and/or a decrease in the value of at least one ratio obtained in step (b) with respect to a cut-off value.

3. The method according to any of claims 1 or 2, **characterized in that** said primers with at least 90% identity with respect to SEQ ID NO: 5-6 are primers with at least 95% identity with respect to SEQ ID NO: 5-6, said primers with at least 90% identity with respect to SEQ ID NO: 8-9 are primers with at least 95% identity with respect to SEQ ID NO: 8-9, and said primers with at least 90% identity with respect to SEQ ID NO: 11-12 are primers with at least 95% identity with respect to SEQ ID NO: 11-12.

4. The method according to claim 3, **characterized in that** said primers with at least 95% identity with respect to SEQ ID NO: 5-6 are primers identified by SEQ ID NO: 5-6, said primers with at least 95% identity with respect to SEQ ID NO: 8-9 are primers identified by SEQ ID NO: 8-9, and said primers with at least 95% identity with respect to SEQ ID NO: 11-12 are primers identified by SEQ ID NO: 11-12.

5. Use of a kit in a method for diagnosing colorectal cancer from a human feces sample according to claim 2, wherein said kit is **characterized in that** it comprises at least one pair of PCR primers selected from the group consisting of SEQ ID NO: 5-6, SEQ ID NO: 8-9 and SEQ ID NO: 11-12.

6. Use of the concentration determined by quantitative PCR in a human feces sample of at least one 16S rDNA bacterial sequence selected from the group consisting of SEQ ID NO: 7 and SEQ ID NO: 10, and/or the value of at least one ratio of concentrations determined by quantitative PCR in said human feces sample selected from the group consisting of SEQ ID NO: 10/SEQ ID NO: 4, and SEQ ID NO: 7/SEQ ID NO: 4, as bacterial marker of the risk of developing colorectal cancer,
wherein SEQ ID NO: 4 concentration is determined using primers with at least 90% identity with respect to SEQ ID NO: 5-6, wherein SEQ ID NO: 7 concentration is determined using primers with at least 90% identity with respect to SEQ ID NO: 8-9, and SEQ ID NO: 10 concentration is determined using primers with at least 90% identity with respect to SEQ ID NO: 11-12; and
wherein an increased risk of developing colorectal cancer is diagnosed if there is an increase in the Ct value of at least one of the sequences of step (a) and/or a decrease in the value of at least one ratio obtained in step (b) with respect to a cut-off value.

7. Use of the concentration determined by quantitative PCR in a human feces sample of at least one 16S rDNA bacterial sequence selected from SEQ ID NO:4, SEQ ID NO:7 and SEQ ID NO:10, and/or the value of at least one ratio of concentrations determined by quantitative PCR in a human feces sample selected from the group consisting of SEQ ID NO: 10/SEQ ID NO: 4, and SEQ ID NO: 7/SEQ ID NO: 4, as bacterial marker of colorectal cancer,
wherein SEQ ID NO: 4 concentration is determined using primers with at least 90% identity with respect to SEQ ID NO: 5-6, SEQ ID NO: 7 concentration is determined using primers with at least 90% identity with respect to SEQ ID NO: 8-9, and SEQ ID NO: 10 concentration is determined using primers with at least 90% identity with respect to SEQ ID NO: 11-12;
wherein colorectal cancer is diagnosed if there is an increase in the Ct value of at least one of the sequences of step (a) and/or a decrease in the value of at least one ratio obtained in step (b) with respect to a cut-off value.

8. The use according to any of claims 6 or 7, **characterized in that** said primers with at least 90% identity with respect to SEQ ID NO: 5-6 are primers with at least 95% identity with respect to SEQ ID NO: 5-6, said primers with at least 90% identity with respect to SEQ ID NO: 8-9 are primers with at least 95% identity with respect to SEQ ID NO: 8-9, and said primers with at least 90% identity with respect to SEQ ID NO: 11-12 are primers with at least 95% identity with respect to SEQ ID NO: 11-12.

9. The use according to claim 8, **characterized in that** said primers with at least 95% identity with respect to SEQ ID NO: 5-6 are primers identified by SEQ ID NO: 5-6, said primers with at least 95% identity with respect to SEQ ID NO: 8-9 are primers identified by SEQ ID NO: 8-9, and said primers with at least 95% identity with respect to SEQ ID NO: 11-12 are primers identified by SEQ ID NO: 11-12.

## Patentansprüche

1. Verfahren zur Diagnose vom Risiko der Entwicklung von Kolorektalkarzinomen aus einer menschlichen Fäkalienprobe, umfassend:
(a) das Bestimmen, mittels quantitativer Polymerasekettenreaktion in der genannten menschlichen Fäkalienprobe, der Konzentration mindestens einer bakteriellen 16S-rDNS-Sequenz, ausgewählt aus der Gruppe bestehend aus SEQ ID NO: 7 und SEQ ID NO: 10,
wobei die SEQ ID NO: 7-Konzentration unter Verwendung von Primern mit mindestens 90% Identität in Bezug auf SEQ ID NO: 8-9 bestimmt wird, und die SEQ ID NO: 10-Konzentration unter Verwendung von Primern mit mindestens 90% Identität in Bezug auf SEQ ID NO: 11-12 bestimmt wird;
(b) wahlweise, das Bestimmen mindestens eines der Konzentrationsverhältnisse, ausgewählt aus der Gruppe bestehend aus SEQ ID NO: 10/SEQ ID NO: 4 und SEQ ID NO: 7/SEQ ID NO: 4, wobei die SEQ ID NO: 4-Konzentration, mittels quantitativer Polymerasekettenreaktion in der genannten menschlichen Fäkalienprobe, unter Verwendung von Primern mit mindestens 90% Identität in Bezug auf SEQ ID NO: 5-6 bestimmt wird; und
(c) das Diagnostizieren des Risikos der Entwicklung von Kolorektalkarzinomen aus der Konzentration mindestens einer der Sequenzen aus Schritt (a) und/oder des Wertes mindestens eines der in Schritt (b) erhaltenen Verhältnisse;
wobei ein erhöhtes Risiko der Entwicklung von Kolorektalkarzinomen in Schritt c) diagnostiziert wird, wenn es eine Erhöhung des Ct-Wertes mindestens einer der Sequenzen aus Schritt (a) und/oder eine Verminderung des Wertes mindestens eines der in Schritt (b) erhaltenen Verhältnisse in Bezug auf einen Grenzwert gibt.

2. Verfahren zur Diagnose von Kolorektalkarzinomen aus einer menschlichen Fäkalienprobe, umfassend:
(a) das Bestimmen, mittels quantitativer Polymerasekettenreaktion in der genannten menschlichen Fäkalienprobe, der Konzentration mindestens einer bakteriellen 16S-rDNS-Sequenz, ausgewählt aus der Gruppe bestehend aus SEQ ID NO: 4, SEQ ID NO: 7 und SEQ ID NO: 10,
wobei die SEQ ID NO: 4-Konzentration unter Verwendung von Primern mit mindestens 90% Identität in Bezug auf SEQ ID NO: 5-6 bestimmt wird, die SEQ ID NO: 7-Konzentration unter Verwendung von Primern mit mindestens 90% Identität in Bezug auf SEQ ID NO: 8-9 bestimmt wird und die SEQ ID NO: 10-Konzentration unter Verwendung von Primern mit mindestens 90% Identität in Bezug auf SEQ ID NO: 11-12 bestimmt wird;
(b) wahlweise, das Bestimmen mindestens eines der Konzentrationsverhältnisse, ausgewählt aus der Gruppe bestehend aus SEQ ID NO: 10/SEQ ID NO: 4 und SEQ ID NO: 7/SEQ ID NO: 4; und
(c) das Diagnostizieren von Kolorektalkarzinomen aus der Konzentration mindestens einer der Sequenzen aus Schritt (a) und/oder des Wertes mindestens eines der in Schritt (b) erhaltenen Verhältnisse;
wobei Kolorektalkarzinomen in Schritt c) diagnostiziert werden, wenn es eine Erhöhung des Ct-Wertes mindestens einer der Sequenzen aus Schritt (a) und/oder eine Verminderung des Wertes mindestens eines der in Schritt (b) erhaltenen Verhältnisse in Bezug auf einen Grenzwert gibt.

3. Verfahren nach einem der Ansprüche 1 oder 2, **dadurch gekennzeichnet, dass** die genannten Primer mit mindestens 90% Identität in Bezug auf SEQ ID NO: 5-6 Primer mit mindestens 95% Identität in Bezug auf SEQ ID NO: 5-6 sind, die genannten Primer mit mindestens 90% Identität in Bezug auf SEQ ID NO: 8-9 Primer mit mindestens 95% Identität in Bezug auf SEQ ID NO: 8-9 sind und die genannten Primer mit mindestens 90% Identität in Bezug auf SEQ ID NO: 11-12 Primer mit mindestens 95% Identität in Bezug auf SEQ ID NO: 11-12 sind.

4. Verfahren nach Anspruch 3, **dadurch gekennzeichnet, dass** die genannten Primer mit mindestens 95% Identität in Bezug auf SEQ ID NO: 5-6 durch SEQ ID NO: 5-6 identifizierte Primer sind, die genannten Primer mit mindestens 95% Identität in Bezug auf SEQ ID NO: 8-9 durch SEQ ID NO: 8-9 identifizierte Primer sind und die genannten Primer mit mindestens 95% Identität in Bezug auf SEQ ID NO: 11-12 durch SEQ ID NO: 11-12 identifizierte Primer sind.

5. Verwendung eines Kits bei einem Verfahren zur Diagnose von Kolorektalkarzinomen aus einer menschlichen Fäkalienprobe nach Anspruch 2, wobei das genannte Kit **dadurch gekennzeichnet ist, dass** es mindestens ein Paar PCR-Primer, ausgewählt aus der Gruppe bestehend aus SEQ ID NO: 5-6, SEQ ID NO: 8-9 und SEQ ID NO: 11-12, umfasst.

6. Verwendung der mittels quantitativer Polymerasekettenreaktion in einer menschlichen Fäkalienprobe mindestens einer bakteriellen 16S-rDNS-Sequenz, ausgewählt aus der Gruppe bestehend aus SEQ ID NO: 7 und SEQ ID NO: 10, bestimmten Konzentration und/oder des Wertes mindestens eines, mittels quantitativer Polymerasekettenreaktion in der genannten menschlichen Fäkalienprobe, ausgewählt aus der Gruppe bestehend aus SEQ ID NO: 10/SEQ ID NO: 4 und SEQ ID NO: 7/SEQ ID NO: 4, bestimmten Konzentrationsverhältnisses als bakterieller Marker des Risikos der Entwicklung von Kolorektalkarzinomen,
wobei die SEQ ID NO: 4-Konzentration unter Verwendung von Primern mit mindestens 90% Identität in Bezug auf SEQ ID NO: 5-6 bestimmt wird, wobei die SEQ ID NO: 7-Konzentration unter Verwendung von Primern mit mindestens 90% Identität in Bezug auf SEQ ID NO: 8-9 bestimmt wird und die SEQ ID NO: 10-Konzentration unter Verwendung von Primern mit mindestens 90% Identität in Bezug auf SEQ ID NO: 11-12 bestimmt wird; und
wobei ein erhöhtes Risiko der Entwicklung von Kolorektalkarzinomen diagnostiziert wird, wenn es eine Erhöhung des Ct-Wertes mindestens einer der Sequenzen aus Schritt (a) und/oder eine Verminderung des Wertes mindestens eines der in Schritt (b) erhaltenen Verhältnisse in Bezug auf einen Grenzwert gibt.

7. Verwendung der mittels quantitativer Polymerasekettenreaktion in einer menschlichen Fäkalienprobe mindestens einer bakteriellen 16S-rDNS-Sequenz, ausgewählt aus SEQ ID NO:4, SEQ ID NO:7 und SEQ ID NO:10, bestimmten Konzentration und/oder des Wertes mindestens eines mittels quantitativer Polymerasekettenreaktion in einer menschlichen Fäkalienprobe, ausgewählt aus der Gruppe bestehend aus SEQ ID NO: 10/SEQ ID NO: 4 und SEQ ID NO: 7/SEQ ID NO: 4, bestimmten Konzentrationsverhältnisses als bakterieller Marker von Kolorektalkarzinomen,
wobei die SEQ ID NO: 4-Konzentration unter Verwendung von Primern mit mindestens 90% Identität in Bezug auf SEQ ID NO: 5-6 bestimmt wird, die SEQ ID NO: 7-Konzentration unter Verwendung von Primern mit mindestens 90% Identität in Bezug auf SEQ ID NO: 8-9 bestimmt wird und die SEQ ID NO: 10-Konzentration unter Verwendung von Primern mit mindestens 90% Identität in Bezug auf SEQ ID NO: 11-12 bestimmt wird;
wobei Kolorektalkarzinomen diagnostiziert werden, wenn es eine Erhöhung des Ct-Wertes mindestens einer der Sequenzen aus Schritt (a) und/oder eine Verminderung des Wertes mindestens eines der in Schritt (b) erhaltenen Verhältnisse in Bezug auf einen Grenzwert gibt.

8. Verwendung nach einem der Ansprüche 6 oder 7, **dadurch gekennzeichnet, dass** die genannten Primer mit mindestens 90% Identität in Bezug auf SEQ ID NO: 5-6 Primer mit mindestens 95% Identität in Bezug auf SEQ ID NO: 5-6 sind, die genannten Primer mit mindestens 90% Identität in Bezug auf SEQ ID NO: 8-9 Primer mit mindestens 95% Identität in Bezug auf SEQ ID NO: 8-9 sind und die genannten Primer mit mindestens 90% Identität in Bezug auf SEQ ID NO: 11-12 Primer mit mindestens 95% Identität in Bezug auf SEQ ID NO: 11-12 sind.

9. Verwendung nach Anspruch 8, **dadurch gekennzeichnet, dass** die genannten Primer mit mindestens 95% Identität in Bezug auf SEQ ID NO: 5-6 durch SEQ ID NO: 5-6 identifizierte Primer sind, die genannten Primer mit mindestens 95% Identität in Bezug auf SEQ ID NO: 8-9 durch SEQ ID NO: 8-9 identifizierte Primer sind und die genannten Primer mit mindestens 95% Identität in Bezug auf SEQ ID NO: 11-12 durch SEQ ID NO: 11-12 identifizierte Primer sind.

## Revendications

1. Procédé pour diagnostiquer le risque de développer un cancer colorectal à partir d'un échantillon de matières fécales humaines, comprenant:
(a) la détermination par PCR quantitative dans ledit échantillon de matières fécales humaines de la concentration d'au moins une séquence bactérienne d'ADNr 16S choisie parmi le groupe composé de SEQ ID NO: 7 et SEQ ID NO: 10,
dans lequel la concentration de SEQ ID NO: 7 est déterminée par l'utilisation d'amorces avec au moins 90% d'identité par rapport à SEQ ID NO: 8-9, et la concentration de SEQ ID NO: 10 est déterminée par l'utilisation d'amorces avec au moins 90% d'identité par rapport à SEQ ID NO: 11-12;
(b) optionnellement, la détermination d'au moins une des proportions des concentrations choisies parmi le groupe composé de SEQ ID NO: 10/SEQ ID NO: 4, et SEQ ID NO: 7/SEQ ID NO: 4, dans lequel la concentration de SEQ ID NO: 4 est déterminée par PCR quantitative dans ledit échantillon de matières fécales humaines en utilisant des amorces avec au moins 90% d'identité par rapport à SEQ ID NO: 5-6; et
(c) le diagnostic du risque de développer un cancer colorectal à partir de la concentration d'au moins une des séquences de l'étape (a) et/ou la valeur d'au moins une proportion obtenue dans l'étape (b);
dans lequel on diagnostique un risque accru de développer un cancer colorectal dans l'étape c) s'il y a une augmentation dans la valeur Ct d'au moins une des séquences de l'étape (a) et/ou une réduction dans la valeur d'au moins une proportion obtenue dans l'étape (b) par rapport à une valeur seuil.

2. Procédé pour diagnostiquer un cancer colorectal à partir d'un échantillon de matières fécales humaines, comprenant:
(a) la détermination par PCR quantitative dans ledit échantillon de matières fécales humaines de la concentration d'au moins une séquence bactérienne d'ADNr 16S choisie parmi le groupe composé de SEQ ID NO: 4, SEQ ID NO: 7 et SEQ ID NO: 10,
dans lequel la concentration de SEQ ID NO: 4 est déterminée par l'utilisation d'amorces avec au moins 90% d'identité par rapport à SEQ ID NO: 5-6, la concentration de SEQ ID NO: 7 est déterminée par l'utilisation d'amorces avec au moins 90% d'identité par rapport à SEQ ID NO: 8-9, et la concentration de SEQ ID NO: 10 est déterminée par l'utilisation d'amorces avec au moins 90% d'identité par rapport à SEQ ID NO: 11-12;
(b) optionnellement, la détermination d'au moins une des proportions des concentrations choisies parmi le groupe composé de SEQ ID NO: 10/SEQ ID NO: 4, et SEQ ID NO: 7/SEQ ID NO: 4; et
(c) le diagnostic d'un cancer colorectal à partir de la concentration d'au moins une des séquences de l'étape (a) et/ou la valeur d'au moins une proportion obtenue dans l'étape (b);
dans lequel on diagnostique un cancer colorectal dans l'étape c) s'il y a une augmentation dans la valeur Ct d'au moins une des séquences de l'étape (a) et/ou une réduction dans la valeur d'au moins une proportion obtenue dans l'étape (b) par rapport à une valeur seuil.

3. Procédé selon l'une quelconque des revendications 1 ou 2, **caractérisé en ce que** lesdites amorces avec au moins 90% d'identité par rapport à SEQ ID NO: 5-6 sont des amorces avec au moins 95% d'identité par rapport à SEQ ID NO: 5-6, lesdites amorces avec au moins 90% d'identité par rapport à SEQ ID NO: 8-9 sont des amorces avec au moins 95% d'identité par rapport à SEQ ID NO: 8-9, et lesdites amorces avec au moins 90% d'identité par rapport à SEQ ID NO: 11-12 sont des amorces avec au moins 95% d'identité par rapport à SEQ ID NO: 11-12.

4. Procédé selon la revendication 3, **caractérisé en ce que** lesdites amorces avec au moins 95% d'identité par rapport à SEQ ID NO: 5-6 sont des amorces identifiées par SEQ ID NO: 5-6, lesdites amorces avec au moins 95% d'identité par rapport à SEQ ID NO: 8-9 sont des amorces identifiées par SEQ ID NO: 8-9, et lesdites amorces avec au moins 95% d'identité par rapport à SEQ ID NO: 11-12 sont des amorces identifiées par SEQ ID NO: 11-12.

5. Utilisation d'une trousse dans un procédé pour diagnostiquer un cancer colorectal à partir d'un échantillon de matières fécales humaines selon la revendication 2, dans lequel ladite trousse est **caractérisée en ce qu'**elle comprend au moins une paire d'amorces de PCR choisies parmi le groupe composé de SEQ ID NO: 5-6, SEQ ID NO: 8-9 et SEQ ID NO: 11-12.

6. Utilisation de la concentration déterminée par PCR quantitative dans un échantillon de matières fécales humaines d'au moins une séquence bactérienne d'ADNr 16S choisie parmi le groupe composé de SEQ ID NO: 7 et SEQ ID NO: 10, et/ou la valeur d'au moins une proportion des concentrations déterminées par PCR quantitative dans ledit échantillon de matières fécales humaines choisie parmi le groupe composé de SEQ ID NO: 10/SEQ ID NO: 4, et SEQ ID NO: 7/SEQ ID NO: 4, comme marqueur bactérien du risque de développer un cancer colorectal,
dans laquelle la concentration de SEQ ID NO: 4 est déterminée par l'utilisation d'amorces avec au moins 90% d'identité par rapport à SEQ ID NO: 5-6, dans laquelle la concentration de SEQ ID NO: 7 est déterminée par l'utilisation d'amorces avec au moins 90% d'identité par rapport à SEQ ID NO: 8-9, et la concentration de SEQ ID NO: 10 est déterminée par l'utilisation d'amorces avec au moins 90% d'identité par rapport à SEQ ID NO: 11-12; et
dans laquelle on diagnostique un risque accru de développer un cancer colorectal s'il y a une augmentation dans la valeur Ct d'au moins une des séquences de l'étape (a) et/ou une réduction dans la valeur d'au moins une proportion obtenue dans l'étape (b) par rapport à une valeur seuil.

7. Utilisation de la concentration déterminée par PCR quantitative dans un échantillon de matières fécales humaines d'au moins une séquence bactérienne d'ADNr 16S choisie parmi SEQ ID NO:4, SEQ ID NO:7 et SEQ ID NO:10, et/ou la valeur d'au moins une proportion des concentrations déterminées par PCR quantitative dans un échantillon de matières fécales humaines choisie parmi le groupe composé de SEQ ID NO: 10/SEQ ID NO: 4, et SEQ ID NO: 7/SEQ ID NO: 4, comme marqueur bactérien du cancer colorectal,
dans laquelle la concentration de SEQ ID NO: 4 est déterminée par l'utilisation d'amorces avec au moins 90% d'identité par rapport à SEQ ID NO: 5-6, la concentration de SEQ ID NO: 7 est déterminée par l'utilisation d'amorces avec au moins 90% d'identité par rapport à SEQ ID NO: 8-9, et la concentration de SEQ ID NO: 10 est déterminée par l'utilisation d'amorces avec au moins 90% d'identité par rapport à SEQ ID NO: 11-12;
dans laquelle on diagnostique un cancer colorectal s'il y a une augmentation dans la valeur Ct d'au moins une des séquences de l'étape (a) et/ou une réduction dans la valeur d'au moins une proportion obtenue dans l'étape (b) par rapport à une valeur seuil.

8. Utilisation selon l'une quelconque des revendications 6 ou 7, **caractérisée en ce que** lesdites amorces avec au moins 90% d'identité par rapport à SEQ ID NO: 5-6 sont des amorces avec au moins 95% d'identité par rapport à SEQ ID NO: 5-6, lesdites amorces avec au moins 90% d'identité par rapport à SEQ ID NO: 8-9 sont des amorces avec au moins 95% d'identité par rapport à SEQ ID NO: 8-9, et lesdites amorces avec au moins 90% d'identité par rapport à SEQ ID NO: 11-12 sont des amorces avec au moins 95% d'identité par rapport à SEQ ID NO: 11-12.

9. Utilisation selon la revendication 8, **caractérisée en ce que** lesdites amorces avec au moins 95% d'identité par rapport à SEQ ID NO: 5-6 sont des amorces identifiées par SEQ ID NO: 5-6, lesdites amorces avec au moins 95% d'identité par rapport à SEQ ID NO: 8-9 sont des amorces identifiées par SEQ ID NO: 8-9, et lesdites amorces avec au moins 95% d'identité par rapport à SEQ ID NO: 11-12 sont des amorces identifiées par SEQ ID NO: 11-12.
